# EUROPÄISCHE PATENTSCHRIFT

(11) **EP 3 315 947 B1**
(45) Veröffentlichungstag und Bekanntmachung des Hinweises auf die Patenterteilung: **07.04.2021**
(21) Anmeldenummer: 17001692.7
(22) Anmeldetag: 13.10.2017
(51) Int. Cl.: G01N 21/3504, A61B 5/08, G01N 33/497, G01N 21/31

(54) **VORRICHTUNG ZUR KONZENTRATIONSBESTIMMUNG MINDESTENS EINER GASKOMPONENTE IN EINEM ATEMGASGEMISCH**
DEVICE FOR DETERMINING THE CONCENTRATION OF AT LEAST ONE GAS COMPONENT IN A BREATHING GAS MIXTURE
DISPOSITIF DE DÉTERMINATION DE LA CONCENTRATION D'AU MOINS UN COMPOSANT DE GAZ DANS UN MÉLANGE DE GAZ RESPIRATOIRE

(30) Priorität: 28.10.2016 DE 102016012970
(43) Veröffentlichungstag der Anmeldung: 02.05.2018
(73) Patentinhaber: Drägerwerk AG & Co. KGaA, 23558 Lübeck (DE)
(72) Erfinder: Dreyer, Peter, 23689 Pansdorf (DE); Steinert, Günter, 23843 Bad Oldesloe (DE); Dicks, Bernd-Michael, 23738 Damlos (DE); Jacobi, Ralph-Peter, 21465 Reinbek (DE)
(74) Vertreter: Meyer-Gramann, Klaus Dieter

(56) Entgegenhaltungen:
- EP-A1- 1 564 545
- EP-A2- 0 196 993
- DE-A1- 10 047 728
- DE-A1-102010 055 182
- DE-U1- 29 602 282
- JP-A- 2009 128 111
- US-A- 5 610 400
- US-A- 5 625 189
- US-A- 5 721 430
- US-A- 6 039 697
- US-A1- 2004 094 154
- US-A1- 2004 203 169
- US-A1- 2012 097 852
- Dexter Research: "ST60 Dual & ST60R Dual", , 2013, Seiten 1-2, XP055441296, Gefunden im Internet: URL:https://www.dexterresearch.com/?module =Resources&event=Download&downloadID=172&f no=1&filename=st60_dual.pdf [gefunden am 2018-01-16]

## Beschreibung

Die Erfindung betrifft eine Analyse-Vorrichtung mit einer Vorrichtung zur Konzentrationsbestimmung einer Gaskomponente in einem Atemgasgemisch. Vorrichtungen zur Konzentrationsbestimmung von Gaskomponenten in einem Atemgasgemisch werden unter anderem eingesetzt, um von Patienten ausgeatmete Konzentrationswerte von Kohlendioxid zu bestimmen.

Die DE10047728 B4 beschreibt einen Sensor zur Messung von Kohlendioxid, Lachgas und Narkosegasen. Es ist eine Detektoranordnung gezeigt, bestehend aus vier optischen Filterelementen mit zugeordneten Detektorelementen. Die Kombinationen aus Filter- und Detektorelement sind um ein Strahlenmischsystem angeordnet. Ein solches Strahlenmischsystem, in Ausführung in einem Multispektralsensor, ist in der EP 0 536 727 B1 gezeigt. Solcherlei Sensorik kommt im klinischen Alltag beispielsweise in einem Kapnographen wie auch einem sog. CO₂-Mainstream-Sensor oder auch einem CO₂-Sidestream-Sensor zum Einsatz. Die US 5 261 415 B2 zeigt einen CO₂-Mainstream-Kapnografie-Sensor. In einer Küvette, welche das Atemgas führt, ist ein Einsatz angeordnet, in welchem wiederum ein Infrarot-optisches Messsystem angeordnet ist. Aus der EP 0 536 727 B1 wird ersichtlich, wie komplex optische Komponenten ausgestaltet und angeordnet werden müssen, um eine wirksame Strahlenmischung zu erreichen. Die Strahlenmischung hat die Aufgabe, lokal auftretende Verschmutzungen symmetrisch sowohl im Referenzkanal wie auch im Messkanal wirksam werden zu lassen. Dies ist erforderlich, um sicherzustellen, dass das Verhältnis von Messkanal zu Referenzkanal in allen Arbeitspunkten derart gewährleistet ist, dass Verschmutzungen, Wasserdampf sowie auch Alterungseffekte der Detektorelemente dauerhaft im Betrieb ausgeglichen werden können. Nachteilig an der Lösung, wie sie in EP 0 536 727 B1 gezeigt ist, ist, dass die Strahlenmischung eine Signalabschwächung dadurch bewirkt, dass das infrarote Licht in der Messküvette mehrfach umgelenkt und reflektiert werden muss. Diese Signalabschwächung führt zu einem schlechteren Signal-Rauschverhältnis (SNR). Um die erforderliche Messwertauflösung zu erreichen, muss eine Erhöhung des Messeffekts mittels einer Erhöhung der Absorptionslänge kompensiert werden. Eine Erhöhung der Absorptionslänge resultiert in einer Vergrößerung der baulichen Ausführung. Das Erfordernis der Strahlmischung und der Vielzahl der daran beteiligten Komponenten wirkt sich weiterhin nachteilig hinsichtlich Komplexität und hoher Toleranzanforderungen der beteiligten Komponenten (Toleranzkette) sowie daraus resultierender hoher Herstellkosten für einen Multispektralsensor vom Typ der EP 0 536 727 B1 aus.

EP 1 564 545 A1 beschreibt eine Vorrichtung zur Konzentrationsbestimmung einer Gaskomponente in einem Einatemgas oder einem Ausatemgas eines Lebewesens mit einer zur Abstrahlung einer Lichtabstrahlung oder Wärmeabstrahlung in einem Wellenlängenbereich von λ1 = 2,5 µm bis λ2 = 14,0 µm geeigneten und ausgestalteten Strahlungsquelle, mit einer Detektoranordnung mit zwei zur Erfassung der von der Strahlungsquelle erzeugten Lichtstrahlung oder Wärmeabstrahlung geeignet ausgebildeten Detektorelementen, mit zwei an den beiden Detektorelementen angeordneten Bandpass-Filterelementen, und mit einer Kontroll-Einheit zur Steuerung des Betriebs der Strahlungsquelle und zur Signalerfassung der zwei Detektorelemente. Eines der Bandpass-Filterelemente ist für infrarote Strahlung optisch durchlässig, die durch das Messgas absorbiert wird. Das andere der beiden Bandpass-Filterelemente ist für Strahlung optisch durchlässig, die durch das Messgas nicht absorbiert wird. Die beiden Detektorelemente liegen mit ihren Detektorflächen in einer Ebene nebeneinander. Gegenüber den beiden Detektorelemeten sind eine Hauptstrahlungsquelle und eine Hilfsstrahlungsquelle nebeneinander liegend angeordnet, die beide auf die nebeneinander liegenden Detektorelemente gerichtet sind. Von der Hauptstrahlungsquelle verläuft ein Hauptstrahlengang zu den Detektorelementen, und von der Hilfsstrahlungsquelle verläuft ein Hilfsstrahlengang zu den Detektorelementen, wobei sowohl Hauptstrahlengang als auch Hilfsstrahlengang nicht senkrecht, sondern unter einem Winkel von mehr als 20° zur Senkrechten auf der Ebene der Detektorflächen der Detektorelemente liegen. Der Hilfsstrahlengang ist dabei für das Messgas nicht zugänglich.

Aus DE 296 02 282 U1 ist ein Infrarot-Gassensor bekannt, der eine Strahlungsquelle, eine Detektoranordnung mit symmetrisch unter einem stumpfen Winkel zur Hauptachse der Abstrahlung der Strahlungsquelle angeordneten Detektorelementen und jeweils davor angeordneten Bandpass-Filterelementen aufweist, von denen eines für Infrarotstrahlung durchlässig ist, die durch das Messgas absorbiert wird, und das andere für Infrarotstrahlung durchlässig ist, die durch das Messgas nicht absorbiert wird. Die Strahlung von der Strahlungsquelle wird jeweils durch einen Lichtwellenleiter zu den Detektorelementen geführt.

US 5,625,189 A beschreibt eine Vorrichtung zur Messung von Absorption von Laserlicht in Atemluft. DE 10 2010 055 182 A1 beschreibt ein Messsystem zur optischen Messung der CO₂-Konzentration in einem Klimaschrank oder einem Inkubator. US 5,610,400 A, US 5,721,430 A und JP 2009 128111 A betreffen Messsensoren für die Infrarot-spektroskopische Analyse von Gasen.

Ausgehend vom vorgenannten Stand der Technik und den dazu beschriebenen Nachteilen ergibt sich als Aufgabe der vorliegenden Erfindung, eine Analyse-Vorrichtung mit einer Vorrichtung zur Gaskonzentrationsbestimmung einer Gaskomponente in einem Atemgasgemisch bereitzustellen, welches sich durch eine einfache, aber effektive Strahlenmischung und einen geringen Platzbedarf bei vergleichsweise günstigen Herstellkosten auszeichnet.

Die Aufgabe wird durch eine Analyse-Vorrichtung mit den Merkmalen des Anspruchs 1 gelöst.

Vorteilhafte Ausführungsformen der Erfindung ergeben sich aus den Unteransprüchen und werden in der folgenden Beschreibung unter teilweiser Bezugnahme auf die Figuren näher erläutert.

Erfindungsgemäß weist die Analyse-Vorrichtung eine Vorrichtung zur Konzentrationsbestimmung einer Gaskomponente in einem Atemgasgemisch eines lebenden Wesens und eine Pumpe auf, die fortlaufend einen Absaugung-Volumenstrom am Mundbereich des Lebewesens über einen Schlauch absaugt und zur Analyse zur Vorrichtung zur Konzentrationsbestimmung abzweigt. Dabei sind *inter alia,* folgende Komponenten in der Vorrichtung zur Konzentrationsbestimmung einer Gaskomponente in einem Atemgasgemisch vorgesehen:
- eine zu einer Abstrahlung einer Lichtabstrahlung oder Wärmeabstrahlung in einem Wellenlängenbereich von lambda1 (λ1) 2,5 µm bis lambda2 (λ2) = 14,0 µm geeignete und ausgestaltete Strahlungsquelle,
- eine Detektoranordnung mit mindestens zwei zur Erfassung der von der Strahlungsquelle erzeugten Lichtabstrahlung oder Wärmeabstrahlung geeignet ausgebildeten Detektorelementen,
- mindestens zwei an den Detektorelementen angeordneten Bandpass-Filterelemente,
- eine Kontrolleinheit.

Die Strahlungsquelle ist als ein mit einer planar ausgestalteten Abstrahlfläche ausgebildeter Flächenstrahler, als ein mit einer planar ausgestalteten Abstrahlfläche ausgebildeter Membranstrahler, als ein mit einer planar ausgestalteten Abstrahlfläche ausgebildetes Strahlungselement oder als eine mit einer planar ausgestalteten Abstrahlfläche ausgebildete Leuchtdiode (LED) ausgebildet, wobei die Abstrahlfläche zu einer über der Abstrahlfläche gleichmäßigen Abstrahlung ausgestaltet ist und die Mittelachse der Abstrahlung senkrecht zur Ebene der Abstrahlfläche steht. Die Abstrahlfläche hat eine Fläche im Bereich von 2,0 mm² bis 10 mm². Das von der Strahlungsquelle abgestrahlte Licht wird also mit einer Mittelachse der Abstrahlung im Wesentlichen senkrecht aus der Abstrahlfläche der Strahlungsquelle in Richtung einer vertikalen Achse der Vorrichtung.

Der Wellenlängenbereich von lambda1 (λ1) = 2,5 µm bis lambda2 (λ2) = 14,0 µm der Strahlungsquelle ermöglicht eine Infrarot-optische Messung von Lachgas-Konzentrationen, Kohlendioxid-Konzentrationen wie auch unterschiedlichen Kohlenwasserstoffen wie beispielsweise volatilen Anästhesiegasen oder Methan.

Mindestens eines der mindestens zwei Bandpass-Filterelemente ist für infrarote Strahlung optisch durchlässig ausgebildet ist, welche durch ein Messgas absorbiert wird, und mindestens eines der mindestens zwei Bandpass-Filterelemente für eine infrarote Strahlung für eine Strahlung optisch durchlässig ausgebildet ist, welche durch das Messgas nicht absorbiert wird.

Zumindest eines der zwei Detektorelemente mit mindestens einem der an den mindestens zwei Detektorelementen angeordneten Bandpass-Filterelementen ist in einer Winkelanordnung mit einem Winkel in einem Bereich von 20° bis 80° zu der Mittelachse der Abstrahlung durch die Strahlungsquelle angeordnet ist.

Dabei ist jedes der mindestens zwei Detektorelemente mit einem ersten Abstand I₁ zur zwischen den mindestens zwei Winkelanordnungen, vorzugsweise mittig verlaufenden vertikalen Mittelachse in einem Bereich von 0,1 mm bis 10 mm angeordnet.

Ferner ist jedes der mindestens zwei an den mindestens zwei Detektorelementen angeordneten Bandpass-Filterelemente mit einem zweiten Abstand I₂ zur zwischen den mindestens zwei Winkelanordnungen verlaufenden vertikalen Mittelachse in einem Bereich von 0,1 mm bis 10 mm angeordnet.

Entweder ist die Detektoranordnung mit den mindestens zwei Detektorelementen und den mindestens zwei an den mindestens zwei Detektorelementen angeordneten Bandpass-Filterelementen gegenüberliegend der Strahlungsquelle in einem dritten Abstand I₃ in einem Bereich von 0,1 mm bis 10,0 mm angeordnet, wobei sich der dritte Abstand I₃ als ein Abstand unmittelbar im Bereich oder entlang der zwischen den mindestens zwei Winkelanordnungen verlaufenden Mittelachse ergibt, wobei die Strahlungsquelle zentrisch auf der zwischen den mindestens zwei Winkelanordnungen verlaufenden Mittelachse zwischen den mindestens zwei Detektorelementen mit den mindestens zwei an den mindestens zwei Detektorelementen angeordneten Bandpass-Filterelementen angeordnet ist; oder alternativ ist die Detektoranordnung mit den mindestens zwei Detektorelementen und den mindestens zwei an den mindestens zwei Detektorelementen angeordneten Bandpass-Filterelementen auf der gleichen Seite benachbart der Strahlungsquelle angeordnet ist, wobei gegenüberliegend der Strahlungsquelle und der mindestens zwei Detektorelemente mit den mindestens zwei an den mindestens zwei Detektorelementen angeordneten Bandpass-Filterelementen in einem dritten Abstand I_{3'} in einem Bereich von 0,1 mm bis 5,0 mm mindestens ein optisch reflektierendes flächig ausgestaltetes Element angeordnet ist, wobei sich der dritte Abstand I_{3'} als ein Abstand unmittelbar im Bereich oder entlang der zwischen den mindestens zwei Winkelanordnungen verlaufenden Mittelachse ergibt.

Die Detektorelemente sind beispielsweise als Halbleiterdetektoren, pyroelektrische Detektoren (Pyrodetektoren), thermoelektrische Detektoren (Thermopiles, Thermoelemente), als thermische Detektoren (Bolometer) wie auch als Kombinationen von Halbleiterdetektoren und thermischen Detektoren ausgestaltet. Die Detektorelemente sind zur Erfassung von infraroter Strahlung in infraroten Wellenlängenbereichen ausgebildet, in welchen typischerweise eine Absorption durch Gase, beispielweise Kohlendioxid, gegeben ist.

Die Bandpass-Filterelemente sind beispielsweise als optische Interferenzfilter in Form von Interferenzschichten auf einem Substrat ausgestaltet. Diese transmittieren Licht in einem definierten Wellenlängenbereich.

Die Anordnung der Bandpass-Filterelemente ist derart ausgestaltet, dass die von der Strahlungsquelle abgestrahlte infrarote Strahlung auf einem direkten Strahlengang oder auch auf einem indirekten Strahlengang, beispielsweise mittels einer Umlenkung der infraroten Strahlung durch reflektive Elemente oder Spiegelanordnungen im Strahlengang, die Bandpass-Filterelemente vor den Detektorelementen passiert. Mindestens eines der mindestens zwei Bandpass-Filterelemente ist für eine infrarote Strahlung in einem Wellenlängenbereich optisch durchlässig ausgebildet, welche durch ein Messgas absorbiert wird.

Das Detektorelement, an dem dieses Bandpass-Filterelement angeordnet ist, stellt den sogenannten Messkanal in der Vorrichtung zur Konzentrationsbestimmung mindestens einer Gaskomponente in einem Atemgasgemisch dar.

Mindestens eines der mindestens zwei Bandpass-Filterelemente ist für eine infrarote Strahlung in einem Wellenlängenbereich optisch durchlässig ausgebildet, welche durch das Messgas nicht absorbiert oder nur geringfügig absorbiert wird.

Das Detektorelement, an dem dieses Bandpass-Filterelement angeordnet ist, stellt den sogenannten Referenzkanal in der Vorrichtung zur Konzentrationsbestimmung mindestens einer Gaskomponente in einem Atemgasgemisch dar.

Messgase, oftmals auch Zielgase genannt, sind beispielsweise Kohlendioxid oder Lachgas wie auch eine Vielzahl gasförmiger, organischer Verbindungen wie Methan oder volatile Anästhesiegase, beispielsweise Halothan, Isofluran, Desfluran, Enfluran.

In der Vorrichtung zur Konzentrationsbestimmung mindestens einer Gaskomponente in einem Atemgasgemisch werden mittels der Kontrolleinheit Messwerte von Messkanal und Referenzkanal erfasst und zueinander in ein Verhältnis gesetzt. Üblicherweise wird ein Quotient aus erfassten Messwerten des Messkanals zu erfassten Messwerten des Referenzkanals gebildet, und dieser Quotient indiziert ein Maß für eine Konzentration des Messgases in der Vorrichtung zur Konzentrationsbestimmung, d.h. der Konzentration einer Gasmenge, welche im Strahlengang befindlich ist.

Die räumliche Anordnung der mindestens zwei Detektorelemente und der Bandpass-Filterelemente in Bezug auf die Strahlungsquelle bzw. in Bezug zur Mittelachse der Abstrahlung erfolgt derart, dass zumindest eines der zwei Detektorelemente mit mindestens einem der an den mindestens zwei Detektorelementen angeordneten Bandpass-Filterelementen in einer Winkelanordnung mit einem Winkel in einem Bereich von 20° bis 80° zu einer durch die Strahlungsquelle hindurch parallel zur Richtung oder identisch mit der senkrecht zur Abstrahlfläche stehenden Mittelachse der Abstrahlung der Strahlungsquelle verlaufenden Achse angeordnet ist.

Die mindestens zwei Detektorelemente mit den daran angeordneten mindestens zwei Bandpass-Filterelementen bilden dabei mindestens zwei Winkelanordnungen zur vertikal aus der Ebene der Abstrahlung der Strahlungsquelle verlaufenden Mittelachse der Abstrahlung.

Die mindestens zwei Winkelanordnungen mit den mindestens zwei Detektorelementen und den an den mindestens zwei Detektorelementen angeordneten Bandpass-Filterelementen bilden zusammen eine Detektoranordnung aus. Die mindestens zwei Winkelanordnungen sind derart im Winkel zur vertikal aus der Ebene der Abstrahlung der Strahlungsquelle verlaufenden Mittelachse der Abstrahlung angeordnet, dass die vertikal aus der Ebene der Abstrahlung der Strahlungsquelle verlaufende Mittelachse zwischen den mindestens zwei Winkelanordnungen verläuft.

Mindestens eine der Winkelanordnungen ist dabei nicht in einer parallelen Ausrichtung zur Ebene der Abstrahlung der Strahlungsquelle angeordnet und ausgestaltet, sondern derart im Winkel zur vertikal aus der Ebene der Abstrahlung der Strahlungsquelle verlaufenden Mittelachse angeordnet, dass die vertikal aus der Ebene der Abstrahlung der Strahlungsquelle verlaufende Mittelachse zwischen den mindestens zwei Winkelanordnungen verläuft und die mindestens eine der Winkelanordnungen zu der vertikal aus der Ebene der Abstrahlung der Strahlungsquelle verlaufenden Mittelachse in einem Winkel im Bereich von 20° bis 80° hin geneigt ist.

In einer bevorzugten Ausführungsform ist mindestens eine der Winkelanordnungen dabei in einer parallelen Ausrichtung zur Ebene der Abstrahlung der Strahlungsquelle angeordnet, dass die vertikal aus der Ebene der Abstrahlung der Strahlungsquelle verlaufende Mittelachse zwischen den mindestens zwei Winkelanordnungen verläuft und die mindestens eine der Winkelanordnungen zu der vertikal aus der Ebene der Abstrahlung der Strahlungsquelle verlaufenden Mittelachse in einem Winkel von 90° rechtwinklig angeordnet ist.

In einer besonderen Ausführungsform sind sämtliche der mindestens zwei Winkelanordnungen derart im Winkel zur vertikal aus der Ebene der Abstrahlung der Strahlungsquelle verlaufenden Mittelachse angeordnet, dass die vertikal aus der Ebene der Abstrahlung der Strahlungsquelle verlaufende Mittelachse zwischen den mindestens zwei Winkelanordnungen verläuft und jede der mindestens zwei Winkelanordnungen jeweils in einem Winkel zu der vertikal aus der Ebene der Abstrahlung der Strahlungsquelle verlaufenden Mittelachse hin geneigt ist. Dabei können die Neigungen der Winkel jeder der mindestens zwei Winkelanordnungen zu der vertikal aus der Ebene der Abstrahlung der Strahlungsquelle zwischen den mindestens zwei Winkelanordnungen verlaufenden Mittelachse voneinander verschieden sein oder nahezu identisch. So sind Ausgestaltungen von 60° für eine der mindestens zwei Winkelanordnungen und von 30° für die andere der mindestens zwei Winkelanordnungen wie auch Ausgestaltungen von 45° für beide der mindestens zwei Winkelanordnungen zur vertikal aus der Ebene der Abstrahlung der Strahlungsquelle zwischen den mindestens zwei Winkelanordnungen verlaufenden Muittelachse möglich.

Diese Ausgestaltung der mindestens zwei Winkelanordnungen bewirkt eine Neigung der mindestens zwei Winkelanordnungen zueinander. Diese Neigung ergibt den Vorteil, dass ein Überlappungsbereich zwischen den Strahlengängen von der Strahlungsquelle zu den Detektorelementen zwischen den mindestens zwei Detektorelementen vorhanden ist.

Dieser Überlappungsbereich ergibt sich lotrecht aus der Ebene der Anordnung der Detektorelemente in Richtung der Strahlungsquelle. Bedingt durch die Winkel befinden sich beispielsweise Gasmoleküle, Wasserdampf, Kondensat oder auch andere Verunreinigungen, beispielsweise Staub, in den Strahlengängen beider Detektorelemente, sodass der Einfluss von Wasserdampf, Kondensat oder auch anderer Verunreinigungen sich im Messsignal beispielsweise als Amplituden-Dämpfung der Messwerte sowohl im Messkanal, wie auch im Referenzkanal niederschlägt. Daraus ergibt sich die Möglichkeit, mittels der Verhältnisbildung der Signale des Referenzkanals und des Messkanals den Einfluss von Feuchtigkeit (Wasserdampf, Kondensat) oder auch anderer Verunreinigungen zu eliminieren. Durch die Wahl der jeweiligen Winkel der Winkelanordnungen von Messkanal-Detektorelement / Bandpassfilterelement und Referenzkanal-Detektorelement / Bandpassfilterelement zueinander wie auch zur zwischen den mindestens zwei Winkelanordnungen verlaufenden Mittelachse kann der Bereich der Überlappung definiert werden.

In Zusammenspiel mit einer Wahl eines vertikalen Abstandes zwischen der Strahlungsquelle und den Winkelanordnungen kann die Ausgestaltung des Überlappungsbereichs mit räumlicher Ausdehnung, flächiger Überlappung, wirksamem Überlappungsvolumen für das Messgas weiterhin variiert und definiert werden.

Durch die zuvor beschriebenen Ausgestaltungen der Winkelanordnungen und des vertikalen Abstandes können in der Vorrichtung zur Konzentrationsbestimmung mindestens einer Gaskomponente in einem Atemgasgemisch in vorteilhafter Weise beispielsweise die Absorptionseigenschaften des zu messenden Messgases berücksichtigt werden, wie auch die gewünschten Konzentrationsmessbereiche des Messgases beeinflusst werden.

Die Bandpass-Filterelemente sind zu einer optischen Filterung von infrarotem Licht in einem Durchlassbereich eines Wellenlängenbereiches von 2,5 µm bis 14 µm ausgestaltet.

Mit solchen Bandpass-Filterelementen sind damit Durchlassbereiche für Gase ermöglicht, wie sie in der nachfolgenden Tabelle 1 aufgeführt sind.

**Tabelle 1**

| Nr. | Gasart | Wellenlängen bereich | |
|---|---|---|---|
| 1 | Kohlendioxid | 4,2 bis 4,4µm | CO₂ |
| | Lachgas | 7,8 bis 9,0µm | N₂O |
| | Methan | 3,1 bis 3,5µm | CH₄ |
| | Ethan | 3,2 bis 3,6µm | C₂H₅ |
| | Halothan | 8 bis 10µm | C₂HBrClF₃ |
| | Isofluran | 8 bis 10µm | C₃H₂OClF₅ |
| | Enfluran | 8 bis 10µm | C₃H₂ClF₅O |
| | Sevofluran | 8 bis 10µm | C₄H₃F₇O |
| | Desfluran | 8 bis 10µm | C₃H₂F₆O |
| | Aceton | 8 bis 10µm | C₃H₆O |
| | Ethanol | 8 bis 10 µm | C₂H₅OH |

Die Gase Lachgas, Halothan, Isofluran, Sevofluran und Desfluran werden während der Durchführung einer Anästhesie, beispielsweise bei chirurgischen Eingriffen, zur Narkotisierung von Patienten eingesetzt, Aceton ergibt sich als ein mögliches Stoffwechselprodukt von Patienten und ist somit beispielsweise in der Ausatemluft von Diabetikern enthalten. Ethanol kann sich beispielsweise bei alkoholisierten Patienten in der Ausatemluft befinden.

Die Detektorelemente sind z.B. als Thermopiles oder Thermoelemente ausgebildet.

Die Detektorelemente sind z.B. als Halbleiterdetektoren, beispielsweise InAsSb-Detektoren (Indium-Arsen-Antimon-Detektoren) ausgebildet.

Die Detektorelemente sind z.B. als Pyrodetektoren ausgebildet.

Die Detektorelemente sind z.B. als Bolometer ausgebildet.

Als Vorteile von Thermoelementen, Thermopiles, Pyrodetektoren und Bolometern sind zu nennen, dass diese kostengünstig herzustellen und als thermische Detektoren in einem weiten Wellenlängenbereich von 3 bis 10µm einsetzbar sind.

Als ein Vorteil von Halbleiterdetektoren ist zu nennen, dass deren Messempfindlichkeit sehr gut auf den gewünschten Wellenlängenbereich abgestimmt werden kann.

In einem Ausführungsbeispiel ist eine Vielzahl von mehr als zwei Detektorelementen mit jeweilig daran angeordneten Bandpass-Filterelementen zu einer kreisförmigen oder rechteckförmigen Anordnung, beispielsweise von Seitenflächen eines Pyramidenstumpfs, zusammen um einen Mittelpunkt angeordnet.

Die Formgebung des Pyramidenstumpfes ist derart, dass dieser gleichsam als ein Trichter eine Form eines über Kopf stehenden Pyramidenstumpfes oder eine Form eines Kleeblattes oder eines Tulpenkelches aufweist. In einer besonderen Variante dieser weiter bevorzugten Ausführungsform weist die Detektoranordnung vier Winkelanordnungen mit Detektorelementen und Bandpass-Filterelementen in einer räumlichen Anordnung in Form von vier Seitenflächen eines rechteckförmigen oder quadratischen Pyramidenstumpfes auf. Auf diese Weise können Messungen mit mehreren Messgasen, beispielsweise mit Hilfe von drei Messkanälen in Bezug zu einem Referenzkanal, erfasst werden.

So sind beispielsweise Messungen von Kohlendioxid (CO₂), Lachgas (N₂O) und einem volatilen Anästhetikum, beispielsweise Halothan (C₂HBrClF₃), Isofluran (C₃H₂OClF₅) in Bezug zum Referenzkanal damit vorteilhaft in einer einzigen Vorrichtung zur Konzentrationsbestimmung mindestens einer Gaskomponente in einem Atemgasgemisch realisierbar.

In einer weiter bevorzugten Ausführungsform bilden in der Vorrichtung zur Konzentrationsbestimmung mindestens einer Gaskomponente in einem Atemgasgemisch die Detektoranordnung und die Strahlungsquelle ein zu einer Führung von Einatemgas und/oder Ausatemgas geeignetes Strömungsführungselement zu einer Strömungsführung in einem Strömungskanal aus. Durch das Strömungsführungselement wird das Einatemgas und/oder Ausatemgas als eine Hauptströmung hindurch geführt und passiert dabei den Strahlengang zwischen der Strahlungsquelle und den mindestens zwei Winkelanordnungen mit den mindestens zwei Detektorelementen und daran angeordneten Bandpass-Filterelementen.

Eine Ausgestaltung einer solchen Ausführungsform ist beispielsweise eine Vorrichtung zu einer Kohlendioxid-Messung im Ausatemgas eines Patienten als Anordnung direkt am Mundbereich des Patienten, welche oftmals auch als ein sogenannter "Mainstream-CO₂-Sensor" bezeichnet wird.

Die Analyse-Vorrichtung dient zur Messung von z.B. Kohlendioxid und weiterer Ausatemgase, insbesondere Anästhesiegase. Die Messung erfolgt im Ausatemgas eines Patienten mittels einer Anordnung, bei welcher direkt am Mundbereich durch eine in der Analyse-Vorrichtung angeordnete Pumpe fortlaufend eine Gasmenge vom Mundbereich über einen Schlauch mit geringem Durchmesser zu der Analyse-Vorrichtung abgesaugt oder gefördert wird und die Gasmenge dort hinsichtlich der Gaszusammensetzung und der Gaskonzentrationen analysiert wird. Eine solche Messmethodik wird oftmals auch als sogenannte "absaugende Gasmessung" oder als sogenanntes "Sidestream-Anästhesiegas- Monitoring" bezeichnet.

Für die Anwendung von Gasmessungen im Umfeld der Anästhesie spielt die Baugröße der Vorrichtung zur Konzentrationsbestimmung mindestens einer Gaskomponente in einem Atemgasgemisch - insbesondere für die "Sidestream"-Anwendung - eine nicht unwichtige Rolle. Der Abstand I₃ in einem vorzugsweisen Bereich von 0,1 mm 10 bis mm zwischen der Strahlungsquelle und den Detektorelementen bzw. Bandpass-Filterelementen wie auch der Abstands I_{3'} im Bereich von 0,1 mm 5,0 bis mm zwischen Strahlungsquelle und dem Reflektorelement (Spiegel) ermöglicht im Zusammenspiel mit den Baugrößen der Strahlungsquelle mit einer Abstrahlfläche im vorzugsweisen Bereich von 2,0 mm² bis 10,0 mm² der Detektorelemente (Bolometer, Mikrobolometer, Mikrobolometer-Arrays, Pyrodetektoren, Thermoelemente, Thermopiles, Halbleiterdetektoren) und Bandpass-Filterelemente mit Flächen in einem vorzugsweisen Bereich von 0,5 mm² bis 20 mm² und der Anordnung der mindestens zwei Detektorelemente zueinander in Abständen in einem vorzugsweisen Bereich kleiner als 10 mm eine Baugröße für die Vorrichtung zur Konzentrationsbestimmung mindestens einer Gaskomponente in einem Atemgasgemisch mit einem geringen Messvolumen in einem Bereich von weniger als 0,4 ml, beispielsweise 0,05 ml bis 0,2 ml.

Bei einer "absaugenden Gasmessung" mit einem Absauge-Volumenstrom mit der in der Vorrichtung angeordneten Pumpe von 50 ml/min bis 200 ml/min ergibt sich dann eine Zeitdauer für einen Austausch des Messvolumens in der Vorrichtung zur Konzentrationsbestimmung mindestens einer Gaskomponente in einem Atemgasgemisch von 0,1 s bis 0,5 s.

Im Vergleich zu Atemfrequenzen von Menschen im Bereich von ungefähr 6 Atemzügen je Minute bis zu 24 Atemzügen je Minute (entsprechend 0,1 bis 0,4 Atemzügen je Sekunde) ermöglicht die mit dieser Erfindung vorgeschlagene Vorrichtung zur Konzentrationsbestimmung mindestens einer Gaskomponente in einem Atemgasgemisch eine zeitliche Messauflösung, die es ermöglicht, in Verbindung mit einer passend gewählten Abtastrate Konzentrationsänderungen im Atemgas als atemzugsaufgelöste Messdaten zu erfassen.

Generell spielt die Baugröße eine weitere wichtige Rolle, da bedingt durch die Abstände nicht nur ein geringes Messvolumen ermöglicht wird, sondern auch die optischen Pfadlängen zwischen den Detektorelementen und der Strahlungsquelle gering gehalten werden können. Das bedingt, dass an den Detektorelemente Messdaten erfassbar sind, welche eine ausreichende Signalhöhe mit einem guten Signal-zu-Rausch-Verhältnis (SNR) aufweisen, sodass eine hohe Messempfindlichkeit in robuster Signalqualität zur Verfügung steht, welche in Verbindung mit passsenden Verstärkerschaltungen und hochwertigen Analog-zu-Digital-Wandlern (A/D-Converter) eine weitgehend rauschfreie hohe Messauflösung, z.B. mit einer 16 Bit- Quantisierung oder feiner (20 Bit, 24 Bit) ermöglichen.

Durch die Überlappung von Messkanal und Referenzkanal wird in vorteilhafter Weise erreicht, dass die atemzugsaufgelösten, erfassten Messdaten frei von Effekten, welche sowohl auf Messkanal wie auch auf den Referenzkanal in ähnlicher Weise einwirken, wie beispielsweise Messgastemperaturänderungen, Verunreinigungen, Wasserdampf, Feuchtigkeit, Verschmutzungen der Strahlungsquelle oder des Reflektorelementes, ohne großen Aufwand einer weiteren Signalverarbeitung und Messdatenkorrektur wie beispielsweise Feuchte- und/oder Temperaturkompensation auf Basis extern bereitgestellter Feuchte- und/oder Temperaturdaten unmittelbar und zeitnah zum Zeitpunkt der tatsächlichen physikalischen Messung zur Verfügung stehen.

Die beschriebenen Ausführungsformen stellen jeweils für sich wie auch in Kombination oder Kombinationen miteinander besondere Ausgestaltungen der Vorrichtung zur Konzentrationsbestimmung von Gaskomponenten in einem Atemgasgemisch dar. Dabei sind auch sämtliche und mögliche sich durch Kombination oder Kombinationen mehrerer Ausführungsformen ergebende weitere Ausführungsformen und deren Vorteile gleichwohl vom Erfindungsgedanken mit erfasst, wenn auch nicht sämtliche Kombinationsmöglichkeiten von Ausführungsformen dazu im Detail jeweils ausgeführt sind.

Die vorliegende Erfindung wird nun mithilfe der folgenden Figuren und den zugehörigen Figurenbeschreibungen ohne Beschränkungen des allgemeinen Erfindungsgedankens näher erläutert. Es zeigen:
- Fig. 1a: eine erste schematische Darstellung einer Vorrichtung zur Konzentrationsbestimmung,
- Fig. 1b: eine weitere, zweite schematische Darstellung einer Vorrichtung zur Konzentrationsbestimmung,
- Fig. 1c: eine schematische Darstellung einer Variante einer Vorrichtung zur Konzentrationsbestimmung nach den Figuren 1a oder 1b,
- Fig. 2: eine Anordnung einer Vorrichtung zur Konzentrationsbestimmung an einem Strömungsführungselement,
- Fig. 3: eine weitere Anordnung einer Vorrichtung zur Konzentrationsbestimmung an einem Strömungsführungselement und
- Fig. 4: ein Strömungsführungselement mit einer Vorrichtung zur Konzentrationsbestimmung.

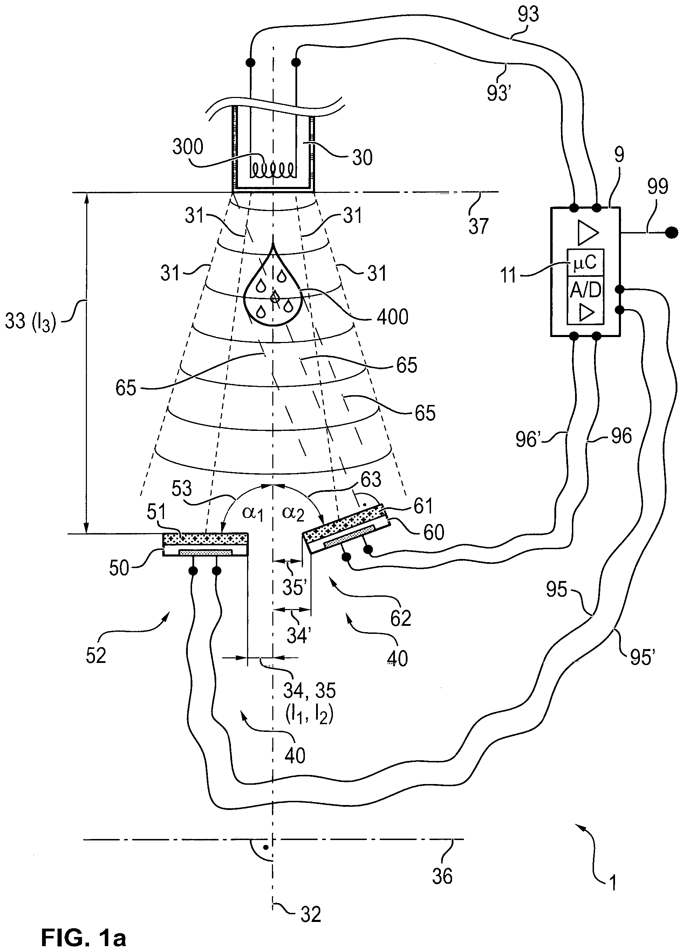

Die Figur 1a zeigt eine erste schematische Darstellung einer Vorrichtung 1 zur Konzentrationsbestimmung mindestens einer Gaskomponente in einem Atemgasgemisch. Die gezeigte Vorrichtung 1 weist eine Strahlungsquelle 30 mit einem Strahlungselement 300 auf. Der Strahlungsquelle 30 gegenüberliegend sind in einem vertikalen Abstand I₃ 33 ein Detektorelement 50 und ein Detektorelement 60 angeordnet. An den Detektorelementen 50, 60 sind Bandpass-Filterelemente 51, 61 angeordnet. Die Bandpass-Filterelemente 51, 61 sind vorzugsweise als Bandpass-Filterelemente ausgeführt, welche einen vorbestimmten Wellenlängenbereich der von der Strahlungsquelle 30 abgestrahlten Strahlung 31 passieren lassen. In dieser Figur 1a ist ein Koordinatensystem mit einer vertikalen Bezugsachse 32 und einer horizontalen Bezugsachse 36 eingezeichnet, auf welches bei der Beschreibung der Lage der Komponenten zueinander und im Hinblick auf die Lage im Raum Bezug genommen wird. So erfolgt eine Abstrahlung 31 von der Strahlungsquelle 30 aus einer horizontalen Ebene der Abstrahlung 37 heraus, wobei die horizontale Ebene 37 parallel zur horizontalen Bezugsebene 36 liegt.

Es ist eine Kontrolleinheit 9 vorgesehen, welche mittels Steuerleitungen 93, 93' mit dem Strahlungselement 300 verbunden ist. Weiterhin ist die Kontrolleinheit 9 mittels Steuerleitungen 96, 96' mit dem Detektorelement 60 verbunden. Weiterhin ist die Kontrolleinheit 9 mittels Steuerleitungen 95, 95' mit dem Detektorelement 50 verbunden. Das Detektorelement 50 mit dem zugehörigen Filterelement 51 bildet zusammen eine Winkelanordnung 52 aus. Das Detektorelement 60 mit dem zugehörigen Filterelement 61 bildet zusammen eine Winkelanordnung 62 aus. Die Winkelanordnungen 52 und 62 ergeben zusammen eine Detektoranordnung 40, welche im Zusammenspiel mit der Strahlungsquelle 30 und der Kontrolleinheit 9 die Vorrichtung 1 zur Konzentrationsbestimmung einer Gaskomponente funktional ausbilden. Die Anordnung der Detektoranordnung 40 in Bezug zur vertikalen Mittelachse 32 und zur horizontalen Bezugsachse 36 wird durch Abstände und Winkel der Winkelanordnungen 52, 62 bestimmt.

Die Winkelanordnung 52 ist in dieser Figur 1a in einer parallelen Anordnung zur horizontalen Bezugsachse 36 wie auch der horizontalen Ebene der Abstrahlung 37 ausgestaltet. Damit ergibt sich ein Winkel α₁ 53 der Winkelanordnung 52 zur vertikalen Mittelachse 32 von 90°. In der Detektoranordnung 40 ergibt sich ein horizontaler Abstand I₁ 34 des Detektorelements 50 zur Mittelachse 32. In der Detektoranordnung 40 ergibt sich ein Abstand I₁ 34' für das Detektorelement 60 zur Mittelachse 32. In der Detektoranordnung 40 ergibt sich ein Abstand I₂ 35 des Bandpass-Filterelementes 51 zur Mittelachse 32. In der Detektoranordnung 40 ergibt sich weiterhin ein Abstand I₂ 35' für das Filterelement 61 zur Mittelachse 32. Bedingt durch die Anordnung der Winkelanordnung 52 in einem Winkel 90° zur Mittelachse 32 sind für das Detektorelement 50 das Filterelement 51 die Abstände I₁ 34 und I₂ 35 zur Mittelachse 32 identisch.

Die Winkelanordnung 62 ist zur Mittelachse 32 in einem Winkel α₂ 63 geneigt ausgeführt. Der Winkel α₂ 63 ist dabei in einem Winkelbereich deutlich kleiner als 90° zur Mittelachse 32 definiert. Bedingt durch die Neigung der Winkelanordnung 62 mit dem Detektorelement 60 und dem Filterelement 61 im Winkel α₂ 63 ergibt sich für die von der Strahlungsquelle 30 abgestrahlte Abstrahlung 31 entlang des vertikalen Abstands I₃ 33 zwischen Strahlungsquelle 30 und Detektoranordnung 40 ein Überlappungsbereich 65 in der Abstrahlung 31. Dieser Überlappungsbereich 65 ergibt sich lotrecht aus der Ebene der Winkelanordnung 62 in Richtung der Strahlungsquelle 30. Bedingt durch die Winkel α₁ 53 und α₂ 63 ergibt sich beispielsweise für Gasmoleküle oder Kondensat (Feuchtigkeit wie Wasserdampf oder Wassertröpfchen) 400, welche in dieser Figur 1a beispielhaft auf der Mittelachse 32 in der Nähe der Strahlungsquelle 30 eingezeichnet sind, die Situation, dass die Abstrahlung 31 der Strahlungsquelle dieses Gasmolekül 400 durchdringt und dabei sowohl als Abstrahlung 31 auf das Detektorelement 50 wie auch auf das Detektorelement 60 wirksam wird. Somit ist sichergestellt, dass beispielsweise Feuchtigkeit (Kondensat) 400 sowohl auf das Detektorelement 50 als auch auf das Detektorelement 60 in gleicher Weise die Abstrahlung dämpfen. Daraus ergibt sich die Möglichkeit, aus der Verhältnisbildung der Signale des Detektorelements 50 und des Detektorelements 60 den Feuchteeinfluss zu eliminieren. Durch die Wahl der Winkel α₁ 53 und α₂ 63 zueinander wie auch zur vertikalen Mittelachse 32 kann der Bereich der Überlappung definiert werden. In Zusammenspiel mit der Wahl des vertikalen Abstandes I₃ 33 zwischen der Strahlungsquelle 30 und der Detektoranordnung 40 wird die Ausdehnung des Überlappungsbereichs 65 weiterhin definiert.

Die Kontrolleinheit 9 wertet die Signale der Detektorelemente 50, 60 mittels geeigneter Elektronikkomponenten 11 (Verstärker, analog zu Digitalwandler, Mikrocontroller) aus und stellt ein Ausgabesignal 99 bereit. Das Ausgabesignal 99 ist dabei repräsentativ für die von den Detektorelementen 50, 60 erfassten Signale wie auch das Verhältnis der erfassten Signale und damit auch repräsentativ für eine aus diesen Signalen bzw. Signalverhältnis abgeleitete Gaskonzentration.

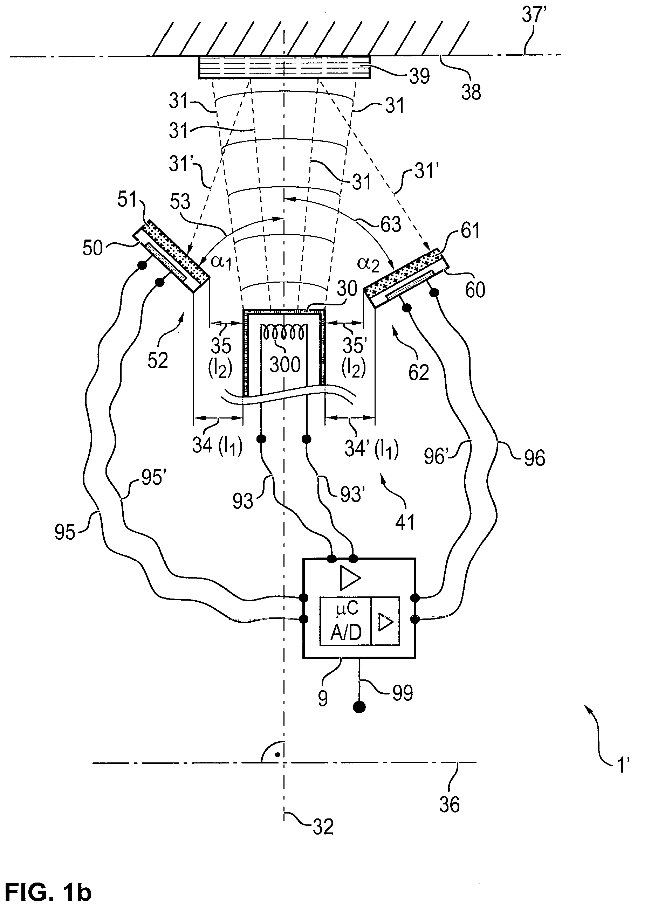

Die Figur 1b zeigt eine weitere, zweite schematische Darstellung einer Vorrichtung 1' zur Konzentrationsbestimmung mindestens einer Gaskomponente in einem Atemgasgemisch. Gleiche Elemente in der Figur 1a und der Figur 1b sind in dieser Figur 1b mit den gleichen Bezugsziffern bezeichnet wie die entsprechend gleichartigen Elemente in der Figur 1a.

Die Figur 1b zeigt mit der weiteren, zweiten schematischen Darstellung eine abgewandelte Variante der Figur 1a. Im Unterschied zu Figur 1a ist in der Fig. 1b die Strahlungsquelle 30 auf der gleichen Seite angeordnet wie die optischen Elemente und die Detektoren. Die gezeigte Vorrichtung 1' weist eine Strahlungsquelle 30 mit einem Strahlungselement 300 auf. Unmittelbar benachbart zur Strahlungsquelle 30 sind ein Detektorelement 50 und ein weiteres Detektorelement 60 angeordnet. An den Detektorelementen 50, 60 sind Bandpass-Filterelemente 51, 61 angeordnet. Der Strahlungsquelle 30 gegenüberliegend ist als eine reflektierende Optik ein Reflektor 39, beispielsweise ein Planspiegel, angeordnet. Der Reflektor 39 wirkt als ein Spiegel für die von der Strahlungsquelle 30 abgestrahlte Strahlung 31 und bewirkt eine Reflexion einer reflektierten Strahlung 31' hin zu den Bandpass-Filterelementen 51, 61 sowie den Detektorelementen 50, 60. Die Bandpass-Filterelemente 51, 61 lassen Licht in einem vorbestimmten Wellenlängenbereich passieren. In dieser Figur 1b ist ein Koordinatensystem mit vertikalen Mittelachsen 32, horizontalen Bezugsachsen 36 eingezeichnet. Diese Achsen dienen in ähnlicher Weise wie in der Beschreibung der Figur 1a als Bezug zur Lage der Komponenten zueinander und im Raum. Es ist eine Kontrolleinheit 9 vorgesehen, welche mit dem Strahlungselement 300 der Strahlungsquelle 30 verbunden ist. Die Anordnung mittels Steuerleitung 93, 93' bzw. 96, 96' sowie 95, 95' zur Verbindung der Kontrolleinheit 9 mit den Detektorelementen 60, 50 entspricht der Anordnung nach Figur 1a und der dazugehörigen Beschreibung, welche hierzu dann in Bezug genommen gelten soll. Das Detektorelement 50 mit dem zugehörigen Filterelement 51 bildet zusammen eine Winkelanordnung 52 aus. Das Detektorelement 60 bildet mit dem zugehörigen Filterelement 61 ebenfalls eine Winkelanordnung 62 aus. Diese Winkelanordnungen 52, 62 ergeben zusammen mit der Strahlungsquelle 30 eine Detektoranordnung 41, welche im Zusammenspiel mit der Kontrolleinheit 9 und dem Reflektor 39 die Vorrichtung 1' zur Konzentrationsbestimmung einer Gaskomponente funktional ausbilden. Die Anordnung der Detektoranordnung 41 in Bezug zu den Achsen 32, 36 wird durch Abstände und Winkel der Winkelanordnung 52, 62 bestimmt. In der Detektoranordnung 41 ergibt sich ein horizontaler Abstand I₁ 34 des Detektorelements 50 zur Mittelachse 32. In der Detektoranordnung 41 ergibt sich ein Abstand I₁ 34' für das Detektorelement 60 zur Mittelachse 32. In der Detektoranordnung 41 ergibt sich ein Abstand I₂ 35 des Bandpass-Filterelementes 51 zur Mittelachse 32. In der Detektoranordnung 41 ergibt sich weiterhin ein Abstand I₂ 35' für das Filterelement 61 zur Mittelachse 32.

Die Winkelanordnungen 52, 62 sind in dieser Figur 1b zur Mittelachse 32 jeweils in Winkeln α₁ 53 und α₂ 63 zur Mittelachse 32 in geneigt ausgeführt.

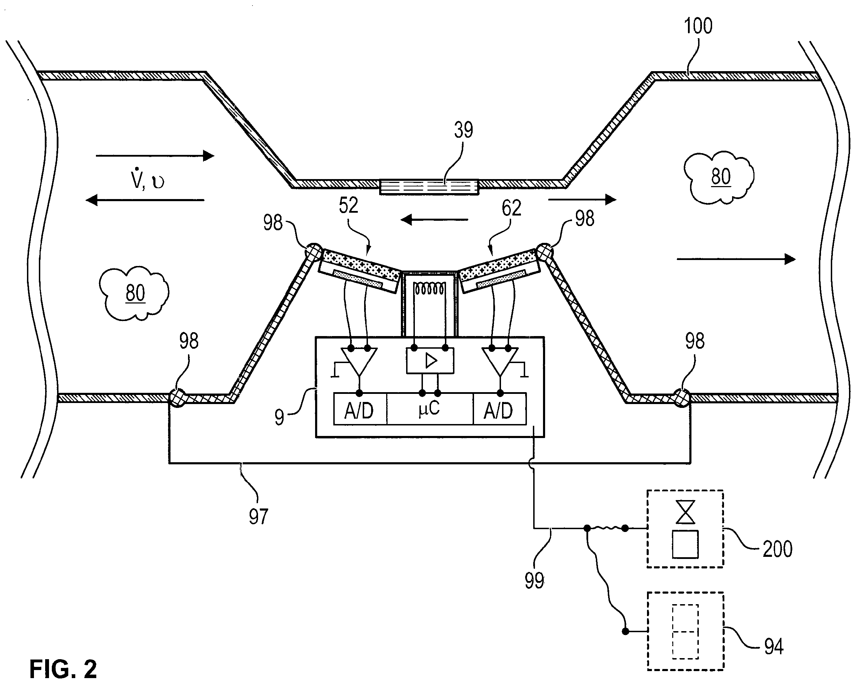

Die Winkel α₁ 53 und α₂ 63 weisen dabei einen Winkelbereich deutlich kleiner als 90° zur Mittelachse 32 auf. Die Winkel α₂ 63 und α₁ 53 sind in dieser Figur 1b beispielhaft mit einem unterschiedlichen Winkelmaß ausgeführt, es ist jedoch vom Gedanken der vorliegenden Erfindung mit umfasst, dass die α₂ 63 und α₁ 53 auch mit einem identischen Winkelmaß zur Mittelachse 32 ausgeführt sein können. Bedingt durch die Neigung der Winkelanordnung 52 mit dem Detektorelement 50 und dem Filterelement 51 im Winkel α₁ 53 und durch die Neigung der Winkelanordnung 62 mit dem Detektorelement 60 und dem Filterelement 61 im Winkel α₂ 63 ergeben sich für die von der Strahlungsquelle 30 abgestrahlte Abstrahlung 31 entlang des vertikalen Abstands zwischen Strahlungsquelle 30 und Detektoranordnung 41 nach Reflexion mittels der Reflektors 39 Überlappungsbereiche in einer reflektierten Strahlung 31'. Die Winkelanordnungen 52, 62 sind in Bezug zur horizontalen Bezugsachse 36, der Mittelachse 32 und zu einer horizontalen Ebene der Lichtreflektion 37', welche parallel zur horizontalen Bezugsachse 36 angeordnet ist, ausgestaltet. Der Überlappungsbereich, welcher sich auf Basis der Winkelanordnungen 52 und 62 ergibt, bedingt, dass Verunreinigungen oder Kondensat, welche beispielsweise in der Nähe des Reflektors 39 in der reflektierten Strahlung 31 vorhanden sind, die Strahlung in gleicher Weise das Detektorelement 50 wie auch das Detektorelement 60 beeinflussen, d. h. ggf. dämpfen. Daraus ergibt sich die Möglichkeit, wie zu Figur 1a beschrieben, aus dem Verhältnis der Signale des Detektorelements 50 und des Detektorelements 60 den Einfluss von Feuchtigkeit 400 (Figur 1a) oder Verunreinigungen zu eliminieren. Der Überlappungsbereich kann durch die Wahl der Winkel α₁ 53 und α₂ 63 zueinander wie auch zur vertikalen Mittelachse 32 definiert werden. Im Unterschied zu Figur 1a ergibt sich in dieser Figur 1b für den Weg der Abstrahlung 31 hin zu dem Reflektor 39 und dem reflektierten Weg der reflektierten Strahlung 31' zu den Detektorelementen 50, 60 ein verlängerter, im einfachsten Fall doppelter Strahlengang. Dies hat zur Folge, dass die auf die Detektorelemente 50, 60 auftreffenden Lichtstrahlen von geringerer Intensität sind als in der Figur 1a. Dies bedingt einen Unterschied hinsichtlich der Empfindlichkeit der Vorrichtung 1' zur Konzentrationsbestimmung einer Gaskomponente in dieser Figur 1b. Die Auswertung der Signale der Detektorelemente 50, 60 in der Kontrolleinheit 9 erfolgt in ähnlicher Weise, wie zu Figur 1a beschrieben, mittels geeigneter Elektronikkomponenten 11. Die Kontrolleinheit stellt ein Ausgabesignal 99 bereit, welches repräsentativ für die Signale der Detektorelemente 50, 60 oder für das Verhältnis der Signale der Detektorelemente 50, 60 ist. Damit stellt das Ausgabesignal 99 eine aus den Signalen abgeleitete Gaskonzentration auf Basis der erfassten Signale der Detektorelemente 50, 60 zu einer weiteren Verarbeitung, beispielsweise einer Anzeigeeinheit 94 (Figur 2) bereit.

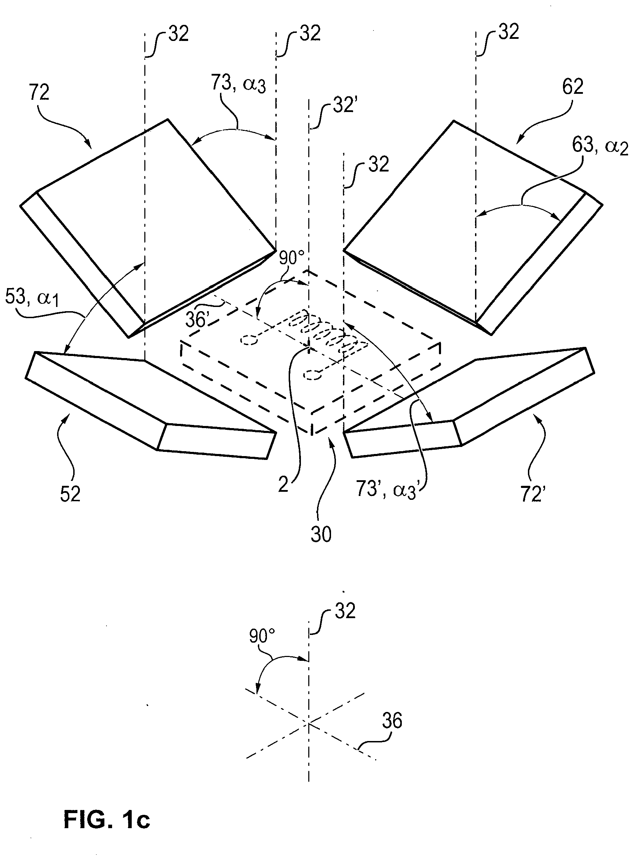

Die Figur 1c zeigt eine schematische Darstellung einer Variante einer Vorrichtung zur Konzentrationsbestimmung nach Figuren 1a oder 1b. Gleiche Elemente in den Figuren 1a, 1b und der Figur 1c sind in dieser Figur 1c mit den gleichen Bezugsziffern bezeichnet wie die entsprechend gleichartigen Elemente in den Figuren 1a und 1b.

Die Figur 1c zeigt eine Weiterbildung nach Figur 1a oder Figur 1b. Die Figuren 1a bzw. 1b zeigen zwei prinzipielle Varianten der Ausgestaltung von Detektorelementen 50, 60, Strahlungsquelle 30 in Verbindung mit oder ohne einen Reflektor 37. Die Figur 1c soll eine Variante zeigen, in welcher nicht nur zwei Detektorelemente 50, 60 als Vorrichtung zur Gasmessung angeordnet sind, sondern insgesamt mehr als zwei Detektorelemente in einer kreisförmigen oder rechteckförmigen Ausgestaltung zueinander arrangiert sind. Eine solche Ausgestaltung mit mehreren Detektorelementen ermöglicht, dass eine Messung mit mehreren Messgasen, beispielsweise von drei oder mehr Gasen, mit drei oder mehr den Messgasen zugeordneten Detektorelementen in Bezug zu einer Referenz mittels eines Referenzdetektorelementes ermöglicht ist. Als geometrische Ausgestaltung ist in dieser Figur 1c ein Trichter, welcher eine Form eines über Kopf stehenden Pyramidenstumpfs mit rechteckiger bzw. quadratischer Grundfläche bzw. Fläche des Stumpfs ausbildet, gezeigt.

Die Figur 1c ist hierzu in folgender Weise ausgestaltet:
Um einen Mittelpunkt 2 herum sind insgesamt vier Winkelanordnungen 52, 62, 72, 72' in zu einer Mittelachse 32 geneigter Anordnung mit Winkeln α1 52, α2 62, α3 73, α3' 73' konstruktiv ausgestaltet. Die horizontalen Achsen 36 sowie 36' und die vertikalen Achsen 32 sind in dieser Figur 1c jeweils an den Winkelanordnungen 52, 62, 72, 72' angeordnet dargestellt, um die räumliche Ausgestaltung in dieser Figur 1c deutlich darstellbar zu machen. Die Achsen 32, 32' 36, 36' verdeutlichen dabei das gleiche räumliche Koordinatensystem wie in den Figuren 1a und 1b.

In einer Ausgestaltung der Figur 1c nach der Figur 1b mit der Vorrichtung 1' zur Konzentrationsbestimmung einer Gaskomponente ist in gestrichelter Linienführung eine Strahlungsquelle 30 mittig zwischen den Winkelanordnungen 52, 62, 72, 72' angeordnet. In dieser Figur 1c nicht gezeichnet, ist gegenüberliegend dieser Strahlungsquelle 30 wiederum ein Reflektorelement erforderlich. Eine solche Ausgestaltung ergibt sich, wie in Figur 1b zu ersehen, mit einer Anordnung eines Reflektors 39 (Figur 1b) an einer der Strahlungsquelle 30 gegenüberliegenden Wandung mit einer horizontalen Ebene der Lichtreflexion 37' (Figur 1b). Die Strahlungsquelle 30 ist hier direkt am Mittelpunkt 2 mit einem Strahlungselement 300, hier als eine Wendel in gestrichelter Linienführung angedeutet, dargestellt.

In einer Ausgestaltung der Figur 1c mit einer Vorrichtung 1 zur Konzentrationsbestimmung einer Gaskomponente nach der Figur 1a entfällt die Strahlungsquelle 30 am Mittelpunkt 2. In einer solchen Ausgestaltung wäre die Strahlungsquelle gegenüberliegend der Winkelanordnungen 52, 62, 72, 72' angeordnet und der Bereich um den Mittelpunkt 2 wäre frei von messtechnischen oder optischen Komponenten (52, 62, 72, 72', 30). Alternativ dazu kann eine Ausgestaltung vorgenommen sein, in welcher dieser Bereich um den Mittelpunkt 2 nicht frei bleibt. Es kann dann eine Ausgestaltung als Variation vorgenommen werden, in welcher dort eine weitere Winkelanordnung ermöglicht ist. Diese weitere Winkelanordnung ist in dieser Figur 1c nicht näher ausgeführt, sie umfasst jedoch alle Merkmale mit einem Detektorelement und einem Filterelement und ist planar zur horizontalen Ebene 36 bzw. 36' als ein Referenzdetektorelement angeordnet. Eine solche weitere Winkelanordnung kann beispielsweise dazu eingesetzt werden, ein Referenzsignal bereitzustellen. Damit ergibt sich dann die Variante, dass vier anstatt der unterschiedlichen drei Gase mit den Winkelanordnungen 52, 62, 72, 72' in Bezug zu dem einem Referenzdetektorelement erfassbar sind, welches mittig zwischen den übrigen vier Detektorelementen angeordnet ist. Damit ergibt sich mittels des Referenzdetektorelementes eine Kompensation von Störungen durch den gemeinsamen - in dieser Figur 1c aus Gründen der Übersichtlichkeit nicht eingezeichneten - Überlappungsbereich 65 (Figur 1a) des Referenzdetektorelementes mit allen vier Winkelanordnungen 52, 62, 72, 72'.

Dies ergibt in vorteilhafter Weise eine Ausführungsform, in welcher Störungen, Verunreinigungen, Kondensat und andere in der Abstrahlung vorhandene Verunreinigungen gleichermaßen für alle drei Messsignale und das Referenzsignal eingehen, so dass eine optimale Kompensation dieser Einflüsse gegeben ist.

Die Figuren 2, 3, 4 zeigen Anordnungen einer Vorrichtung zur Konzentrationsbestimmung nach den Figuren 1a, 1b, 1c an einem Strömungsführungselement. Die Figuren 2, 3, 4 sollen in einer gemeinsamen Figurenbeschreibung hinsichtlich der gemeinsamen Merkmale miteinander, aber auch hinsichtlich der Unterschiede zueinander, beschrieben werden.

Gleiche Elemente in den Figuren 2, 3, 4 und den Figuren 1a, 1b, 1c sind mit den gleichen Bezugsziffern bezeichnet wie die entsprechend gleichartigen Elemente in den Figuren 2, 3, 4 wie in den Figuren 1a, 1b, 1c.

In der Figur 2 ist eine Vorrichtung 1' zur Konzentrationsbestimmung einer Gaskomponente (Figur 1b) in einem Strömungsführungselement 100 gezeigt. Das Strömungsführungselement 100 ist ausgestaltet, um eine Strömung mit einer Gasmenge 80 einer Messung mittels der Vorrichtung 1' (Figur 1b) zuzuführen. Es sind Winkelanordnungen 52, 62 in Verbindung mit einer Strahlungsquelle, einem Strahlungselement und einer Kontrolleinheit 9 gezeigt. Die Winkelanordnungen 52, 62 mit der Strahlungsquelle und der Kontrolleinheit 9 sind in einem Bauteil 97 angeordnet, welches mittels Dichtelementen 98 an das Strömungsführungselement 100 angekoppelt ist. Die Funktionsweise der Anordnung nach Figur 2 ergibt sich wie zu Figur 1b beschrieben.

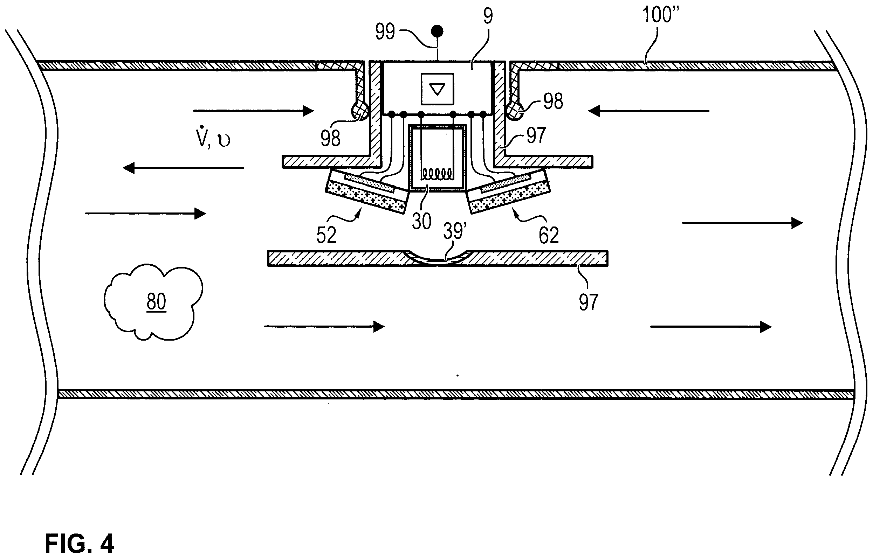

In der Figur 4 ist eine zu der Figur 2 vergleichbare Anordnung in einem Strömungskanal 100" mit einer Vorrichtung 1' zur Konzentrationsbestimmung einer Gaskomponente gezeigt. Hier ist ein Bauteil 97 vorhanden, welches mittels Dichtelementen 98 in das Strömungsführungselement 100" eingesetzt ist. In dem Strömungsführungselement 100" gelangt im Unterschied zu Figur 2 in dieser Figur 4 nur eine Teilmenge in Form einer Nebenströmung der in dem Strömungsführungselement 100" strömenden Gasmenge in die Vorrichtung zur Konzentrationsbestimmung einer Gaskomponente 1' (Figur 1b). Diese Figur 4 stellt somit eine Messung in einem sogenannten Bypass dar. In dieser Figur 4 ist gegenüberliegend der Strahlungsquelle 30 wie auch in Figur 2 ein Reflektor 39', diesmal in gewölbter Ausführung gezeigt, als Teil des Bauteils 97 angeordnet.

In dieser Figur 4 ist die Vorrichtung 1' (Figur 1b) zur Konzentrationsbestimmung einer Gaskomponente in einer Neben- oder Seitenströmung in dem Bypass mit der Anbringung des Bauteils 97 als Einsatz in das Strömungsführungselementes 100" hinein - ausgestaltet in Form eines "T-Stückes" - nahezu in der Strömungsmitte des Strömungsführungselementes 100", also im Wesentlichen im Zentrum des Strömungsführungselementes 100", angeordnet und messtechnisch wirksam. Alternativ und zusätzlich wird mit dieser Figur 4 auch eine Anordnung des Bauteils 97 veranschaulicht, wobei die Vorrichtung 1' (Figur 1b) zur Konzentrationsbestimmung einer Gaskomponente nicht in der Strömungsmitte, sondern im Randbereich des Strömungsführungselementes 100" angeordnet und messtechnisch wirksam ist. Damit ergibt sich eine Ausgestaltung des Bypass' im Bereich einer Randströmung im Randbereich des Strömungsführungselementes 100".

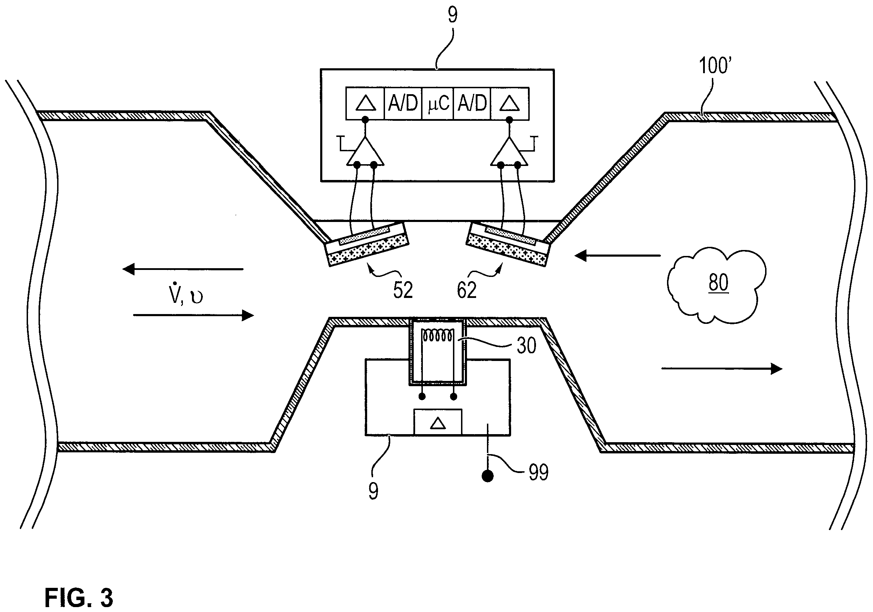

In der Figur 3 ist im Unterschied zu Figur 2 wie auch zu Figur 4 eine Vorrichtung 1 zur Konzentrationsbestimmung einer Gaskomponente gemäß der Figur 1a in einem Strömungskanal 100' gezeigt. Die Strahlungsquelle 30 ist gegenüberliegend von zwei Winkelanordnungen 52, 62 an einem Strömungsführungselement 100' angeordnet. Die Anordnung der Winkelanordnungen 52, 62 und der Strahlungsquelle 30 erfolgt gegenüberliegend an einer Stelle des Strömungsführungselements 100, an der der Strömungsquerschnitt in Form eines Venturi-Rohres verringert ist. In dieser Ausgestaltung nach Figur 3 ist es erforderlich, von zwei Seiten Elemente einer Kontrolleinheit 9 vorzusehen. Dadurch wird ermöglicht, sowohl die Winkelanordnungen 52, 62 mit den Detektorelementen 50, 60 (Figur 1a) zu betreiben und die Signale zu verstärken. Zusätzlich dient die Kontrolleinheit 9 zur Ansteuerung der Strahlungsquelle 30 und zur Ausgabe eines Ausgabesignals 99.

In den Figuren 2, 3, 4 ist ein Ausgabesignal 99 vorgesehen, welches, wie zuvor in Figur 1a und 1b ausgeführt, repräsentativ für eine erfasste Gaskonzentration ist.

Die Figur 3 wie auch die Figur 2 sind im Unterschied zu Figur 4 so ausgeführt, dass die Messung der Gaskonzentration der Gasmenge 80 nicht in einem Bypassstrom, sondern in der Hauptströmung direkt erfolgt. In der Figur 2 sind ein Medizingerät 200 sowie eine Anzeigeeinheit 94 jeweils als optionale Komponenten in gestrichelter Linienführung eingezeichnet. Diese optionalen Komponenten stellen beispielhafte Möglichkeiten dar, das Ausgabesignal 99 einer weiteren Verarbeitung und Verwendung zuzuführen.

In den Figuren 3 und 4 sind diese optionalen Komponenten 200, 94 nicht gezeigt, sollen aber gleichwohl mit vom Erfindungsgedanken auch in die Ausgestaltungen nach diesen Figuren 3 und 4 mit einbezogen gelten.

### Bezugszeichen liste

- 1,1': Vorrichtung zur Konzentrationsbestimmung einer Gaskomponente
- 2: Mittelpunkt (Schnittpunkt der Achsen 32' und 36')
- 9: Kontrolleinheit
- 11: Elektronik-Komponenten
- 30: Strahlungsquelle
- 31: Abstrahlung
- 31': reflektierte Strahlung
- 32, 32': vertikale Achse, Mittelachse,
- 33: I_{3'} I_{3'} vertikaler Abstand
- 34: I₁ Abstand des Detektorelements 50 zur Mittelachse 32
- 34': I₁ Abstand des Detektorelements 60 zur Mittelachse 32
- 35: I₂ Abstand des Filterelements 51 zur Mittelachse 32
- 35': I₂ Abstand des Filterelements 61 zur Mittelachse 32
- 36, 36': horizontale Bezugsachse
- 37: horizontale Ebene der Abstrahlung
- 37': horizontale Ebene der Lichtreflexion
- 38: Wandung
- 39, 39': Reflektor, Spiegelelement
- 40: Detektoranordnung
- 41: Detektoranordnung, reflektiv
- 50: Detektorelement
- 51: Bandpass-Filterelement
- 52: Winkelanordnung
- 53: Winkel α₁
- 60: Detektorelement
- 61: Bandpass-Filterelement
- 62: Winkelanordnung
- 63: Winkel α₂
- 65: Überlappungsbereich
- 72: Winkelanordnung
- 72': Winkelanordnung
- 73: Winkel α₃
- 73': Winkel α_{3'}
- 80: Gasmenge, Gaskonzentration
- 93, 93': Steuerleitung zum Strahlungselement 300
- 94: Anzeigeeinheit
- 95, 95': Datenleitung, Signalleitung
- 96, 96': Datenleitung, Signalleitung
- 97: Bauteil
- 98: Einsatz, Dichtelement
- 99: Ausgabesignal
- 100, 100', 100": Strömungsführungselement
- 200: Medizingerät, Beatmungsgerät, Anästhesiegerät
- 300: Strahlungselement, (Membran, Wendel)
- 400: Gasmolekül, Kondensat

## Patentansprüche

1. Analyse-Vorrichtung mit einer Vorrichtung (1,1') zur Konzentrationsbestimmung einer Gaskomponente bzw. eines Messgases in einem Atemgasgemisch eines Lebewesens, und mit einer Pumpe, die eingerichtet ist, fortlaufend einen Absaug-Volumenstrom am Mundbereich des Lebewesens über einen Schlauch abzusaugen und zur Analyse zur Vorrichtung zur Konzentrationsbestimmung abzuzweigen,
wobei die Vorrichtung zur Konzentrationsbestimmung versehen ist
- mit einer zu einer Abstrahlung (31) einer Lichtabstrahlung oder Wärmeabstrahlung in einem Wellenlängenbereich von lambda1 (λ1) = 2,5 µm bis lambda2 (λ2) = 14,0 µm geeigneten und ausgestalteten Strahlungsquelle (30), wobei die Strahlungsquelle (30) als ein mit einer planar augestalteten Abstrahlfläche ausgebildeter Flächenstrahler, als ein mit einer planar augestalteten Abstrahlfläche ausgebildeter Membranstrahler, als ein mit einer planar ausgestalteten Abstrahlfläche ausgebildetes Strahlungselement (300) oder als eine mit einer planar ausgestalteten Abstrahlfläche ausgebildete Leuchtdiode, LED, ausgebildet ist, wobei die Abstrahlfläche zu einer über der Abstrahlfläche gleichmäßigen Abstrahlung (31) ausgestaltet ist, wobei eine Bezugsachse (32) in Richtung der Abstrahlung, im folgenden Mittelachse (32) der Abstrahlung genannt, senkrecht zur Ebene (37) der Abstrahlfläche steht und wobei die Abstrahlfläche eine Fläche im Bereich von 2,0 mm² bis 10 mm² hat,
- mit einer Detektoranordnung (40; 41) mit mindestens zwei zu einer Erfassung der von der Strahlungsquelle (30) erzeugten Lichtabstrahlung oder Wärmeabstrahlung geeignet ausgebildeten Detektorelementen (50, 60),
- mit mindestens zwei an den mindestens zwei Detektorelementen (50, 60) angeordneten Bandpass-Filterelementen (51, 61),
- mit einer Kontroll-Einheit (9) zu einer Steuerung eines Betriebs der Strahlungsquelle (30) und zu einer Signalerfassung der mindestens zwei Detektorelemente (50, 60),
wobei mindestens eines der mindestens zwei Bandpass-Filterelemente (51, 61) für eine infrarote Strahlung optisch durchlässig ausgebildet ist, welche durch ein Messgas absorbiert wird,
wobei mindestens eines der mindestens zwei Bandpass-Filterelemente (51, 61) für eine infrarote Strahlung für eine Strahlung optisch durchlässig ausgebildet ist, welche durch das Messgas nicht absorbiert wird,
wobei zumindest eines der zwei Detektorelemente (50, 60) mit mindestens einem der an den mindestens zwei Detektorelementen (50, 60) angeordneten Bandpass-Filterelementen (51, 61) in einer Winkelanordnung (52, 62) mit einem Winkel (53, 63) in einem Bereich von 20° bis 80° zu der Mittelachse (32) der Abstrahlung durch die Strahlungsquelle (30) angeordnet ist,
wobei jedes der mindestens zwei Detektorelemente (50, 60) mit einem ersten Abstand I₁ (34, 34') zur zwischen den mindestens zwei Winkelanordnungen (52, 62) verlaufenden Mittelachse (32) in einem Bereich von 0,1 mm bis 10,0 mm angeordnet ist, wobei jedes der mindestens zwei an den mindestens zwei Detektorelementen (50, 60) angeordneten Bandpass-Filterelemente (51, 61) mit einem zweiten Abstand I₂ (35) zur zwischen den mindestens zwei Winkelanordnungen (52, 62) verlaufenden Mittelachse (32) in einem Bereich von 0,1 mm bis 10,0 mm angeordnet ist,
wobei
(i) entweder die Detektoranordnung (41) mit den mindestens zwei Detektorelementen (50, 60) und den mindestens zwei an den mindestens zwei Detektorelementen (50, 60) angeordneten Bandpass-Filterelementen (51, 61) gegenüberliegend der Strahlungsquelle (30) in einem dritten Abstand I₃ (33) in einem Bereich von 0,1 mm bis 10,0 mm angeordnet ist, wobei sich der dritte Abstand I₃ (33) als ein Abstand unmittelbar im Bereich oder entlang der zwischen den mindestens zwei Winkelanordnungen (52, 62) verlaufenden Mittelachse (32) ergibt, wobei die Strahlungsquelle (30) zentrisch auf der zwischen den mindestens zwei Winkelanordnungen (52, 62) verlaufenden Mittelachse (32) zwischen den mindestens zwei Detektorelementen (50, 60) mit den mindestens zwei an den mindestens zwei Detektorelementen (50, 60) angeordneten Bandpass-Filterelementen (51, 61) angeordnet ist,
oder
(ii) die Detektoranordnung (40) mit den mindestens zwei Detektorelementen (50, 60) und den mindestens zwei an den mindestens zwei Detektorelementen (50, 60) angeordneten Bandpass-Filterelementen (51, 61) auf der gleichen Seite benachbart der Strahlungsquelle (30) angeordnet ist, wobei gegenüberliegend der Strahlungsquelle (30) und der mindestens zwei Detektorelemente (50, 60) mit den mindestens zwei an den mindestens zwei Detektorelementen (50, 60) angeordneten Bandpass-Filterelementen (51, 61) in einem dritten Abstand I_{3'} in einem Bereich von 0,1 mm bis 5,0 mm mindestens ein optisch reflektierendes flächig ausgestaltetes Element (39) angeordnet ist, wobei sich der dritte Abstand I_{3'} als ein Abstand unmittelbar im Bereich oder entlang der zwischen den mindestens zwei Winkelanordnungen (52, 62) verlaufenden Mittelachse (32) ergibt;
und wobei die Pumpe eingerichtet ist, den Volumenstrom fortlaufend dem Strahlengang von der Strahlungsquelle (30) zur Detektoranordnung (40; 41) zuzuführen.

2. Analyse-Vorrichtung nach Anspruch 1, wobei die Bandpass-Filterelemente (51, 61, 71, 81) zu einer optischen Filterung von infrarotem Licht in einem Durchlassbereich eines Wellenlängenbereiches von 2,5 µm bis 14 µm ausgestaltet sind.

3. Analyse-Vorrichtung nach einem der vorherigen Ansprüche, wobei die Detektorelemente (50, 60) als Pyrodetektoren, Bolometer, Halbleiterdetektoren, Thermopiles oder Thermoelemente ausgestaltet sind.

4. Analyse-Vorrichtung nach einem der vorherigen Ansprüche, wobei die Detektoranordnung (40) mehr als zwei Winkelanordnungen (52, 62, 72, 72') mit Detektorelementen und Bandpass-Filterelementen in einer räumlichen Anordnung in Form von Seitenflächen eines rechteckförmigen oder quadratischen Pyramidenstumpfes um einen Mittelpunkt (2) aufweist.

5. Analyse-Vorrichtung nach einem der Ansprüche 1 bis 4, wobei die Detektor-Anordnung (40, 41) zusammen mit der Strahlungsquelle (30) und, falls vorgesehen, dem optisch reflektierenden Element (39) ein zu einer Führung des Atemgasgemisches geeignetes Strömungsführungselement ausbilden, so dass das Atemgasgemisch das Strömungsführungselement als Strömung durchströmt und die Gaskonzentration in der Strömung erfassbar ist.

6. Analyse-Vorrichtung nach einem der Ansprüche 1 bis 4, umfassend ein Strömungsführungselement (100, 100') geeignet zur Führung des Atemgasgemisches, an welchem die Detektoranordnung (40) zusammen mit der Strahlungsquelle (30) und, falls vorgesehen, dem reflektierenden Element (39) derart angeordnet sind, so dass Atemgasgemisch zur Konzentrationsmessung verwendet wird, welches im Wesentlichen in der Mitte des Strömungsführungselement (100) als Hauptströmung hindurch strömt.

7. Vorrichtung (1) nach einem der Ansprüche 1 bis 4, umfassend ein Strömungsführungselement (100") geeignet zur Führung des Atemgasgemisches, wobei die Detektoranordnung (41) zusammen mit der Strahlungsquelle (30) und, falls vorgesehen, dem reflektierenden Element (39) als ein seitlich angeordnetes Bauteil (97) in dem Strömungsführungselement (100") ausgebildet sind, wobei das Bauteil (97) derart ausgebildet ist, dass Atemgasgemisch zur Konzentrationsmessung verwendet wird, welches im Wesentlichen in einem seitlichen Randbereich des Strömungsführungselementes (100") in einer Nebenströmung als ein Teil einer Hauptströmung durch das Bauteil hindurch strömt.

## Claims

1. Analysis device comprising a device (1, 1') for determining a concentration of a gas component or of a measurement gas in a respiratory gas mixture of a living being, and comprising a pump configured to continuously extract by suction an extraction volume flow at the mouth region of the living being via a tube and to divert it to the concentration determining device for analysis, wherein the concentration determining device is provided
- with a radiation source (30) configured and suitable for an emission (31) of a light emission or heat emission in a wavelength range of lambda 1 (λ1) = 2.5 µm to lambda 2 (λ2) = 14.0 µm, wherein the radiation source (30) is embodied as a surface radiator embodied with an emission surface configured in a planar fashion, as a membrane radiator embodied with an emission surface configured in a planar fashion, as a radiation element (300) embodied with an emission surface configured in a planar fashion, or as a light-emitting diode, LED, embodied with an emission surface configured in a planar fashion, wherein the emission surface is configured for an emission (31) that is uniform over the emission surface, wherein a reference axis (32) in the direction of the emission, referred to hereinafter as centre axis (32) of the emission, is perpendicular to the plane (37) of the emission surface and wherein the emission surface has an area in the range of 2.0 mm² to 10 mm²,
- with a detector arrangement (40; 41) comprising at least two detector elements (50, 60) embodied suitably for detecting the light emission or heat emission generated by the radiation source (30),
- with at least two bandpass filter elements (51, 61) arranged at the at least two detector elements (30, 60),
- with a control unit (9) for controlling operation of the radiation source (30) and for acquiring signals of the at least two detector elements (30, 60),
wherein at least one of the at least two bandpass filter elements (51, 61) is embodied as optically transmissive for infrared radiation that is absorbed by a measurement gas,
wherein at least one of the at least two bandpass filter elements (51, 61) is embodied as optically transmissive for infrared radiation for a radiation that is not absorbed by the measurement gas, wherein at least one of the two detector elements (50, 60) with at least one of the bandpass filter elements (51, 61) arranged at the at least two detector elements (50, 60) is arranged in an angular arrangement (52, 62) at an angle (53, 63) in a range of 20° to 80° with respect to the centre axis (32) of the emission through the radiation source (30), wherein each of the at least two detector elements (50, 60) is arranged at a first distance I₁ (34, 34') from the centre axis (32), running between the at least two angular arrangements (52, 62) in a range of 0.1 mm to 10.0 mm,
wherein each of the at least two bandpass filter elements (51, 61) arranged at the at least two detector elements (50, 60) is arranged at a second distance I₂ (35) from the centre axis (32), running between the at least two angular arrangements (52, 62) in a range of 0.1 mm to 10.0 mm,
wherein
(i) either the detector arrangement (41) with the at least two detector elements (50, 60) and the at least two bandpass filter elements (51, 61) arranged at the at least two detector elements (50, 60) is arranged opposite the radiation source (30) at a third distance I₃ (33) in a range of 0.1 mm to 10.0 mm, wherein the third distance I₃ (33) is obtained as a distance directly in the region of or along the centre axis (32) running between the at least two angular arrangements (52, 62), wherein the radiation source (30) is arranged centrally on the centre axis (32), running between the at least two angular arrangements (52, 62), between the at least two detector elements (50, 60) with the at least two bandpass filter elements (51, 61) arranged at the at least two detector elements (50, 60),
or
(ii) the detector arrangement (40) with the at least two detector elements (50, 60) and the at least two bandpass filter elements (51, 61) arranged at the at least two detector elements (50, 60) is arranged on the same side adjacent to the radiation source (30), wherein at least one optically reflective element (39) configured in an areal fashion is arranged opposite the radiation source (30) and the at least two detector elements (50, 60) with the at least two bandpass filter elements (51, 61) arranged at the at least two detector elements (50, 60) at a third distance I_{3'} in a range of 0.1 mm to 5.0 mm, wherein the third distance I_{3'} is obtained at a distance directly in the region of or along the centre axis (32) running between the at least two angular arrangements (52, 62);
and wherein the pump is configured to feed the volume flow continuously to the beam path from the radiation source (30) to the detector arrangement (40; 41).

2. Analysis device according to Claim 1, wherein the bandpass filter elements (51, 61, 71, 81) are configured for optically filtering infrared light in a passband of a wavelength range of 2.5 µm to 14 µm.

3. Analysis device according to either of the preceding claims, wherein the detector elements (50, 60) are configured as pyrodetectors, bolometers, semiconductor detectors, thermopiles or thermocouples.

4. Analysis device according to any of the preceding claims, wherein the detector arrangement (40) has more than two angular arrangements (52, 62, 72, 72') with detector elements and bandpass filter elements in a spatial arrangement in the form of side surfaces of a rectangular or square truncated pyramid around a centre point (2).

5. Analysis device according to any of Claims 1 to 4, wherein the detector arrangement (40, 41) together with the radiation source (30) and, if provided, the optically reflective element (39) form a flow guide element suitable for guiding the respiratory gas mixture, such that the respiratory gas mixture flows through the flow guide element as a flow and it is possible to detect the gas concentration in the flow.

6. Analysis device according to any of Claims 1 to 4, comprising a flow guide element (100, 100') suitable for guiding the respiratory gas mixture, at which the detector arrangement(40) together with the radiation source (30) and, if provided, the reflective element (39) are arranged in such a way that respiratory gas mixture, which flows through substantially in the centre of the flow guide element (100) as a main flow, is used for the concentration measurement.

7. Device (1) according to any of Claims 1 to 4, comprising a flow guide element (100") suitable for guiding the respiratory gas mixture, wherein the detector arrangement (41) together with the radiation source (30) and, if provided, the reflective element (39) are embodied as a laterally arranged component (97) in the flow guide element (100"), wherein the component (97) is embodied in such a way that respiratory gas mixture, which flows through the component substantially in a lateral edge region of the flow guide element (100") in a secondary flow as a part of a main flow, is used for the concentration measurement.

## Revendications

1. Dispositif d'analyse, comprenant un dispositif (1, 1') permettant de déterminer la concentration d'un composant de gaz ou d'un gaz de mesure dans un mélange de gaz respiratoire d'un sujet, et une pompe qui est aménagée pour aspirer en continu un débit volumique d'aspiration au niveau de la bouche du sujet par une tubulure et pour le détourner vers le dispositif de détermination de concentration en vue d'une analyse, dans lequel le dispositif de détermination de concentration est doté
- d'une source de rayonnement (30) adaptée et configurée pour émettre (31) un rayonnement de lumière ou un rayonnement de chaleur dans une gamme de longueurs d'onde de lambda1 (λ1) = 2,5 µm à lambda2 (λ2) = 14,0 µm, la source de rayonnement (30) étant réalisée sous la forme d'un radiateur à surface plane réalisé avec une surface de rayonnement à configuration plane, sous la forme d'un radiateur à membrane réalisé avec une surface de rayonnement de configuration plane, sous la forme d'un élément radiant (300) réalisé avec une surface de rayonnement à configuration plane, ou sous la forme d'une diode électroluminescente, LED, réalisée avec une surface de rayonnement à configuration plane, la surface de rayonnement étant configurée pour un rayonnement (31) homogène sur la surface de rayonnement, dans lequel un axe de référence (32) en direction du rayonnement, appelé par la suite axe médian (32) du rayonnement, est perpendiculaire au plan (37) de la surface de rayonnement, et la surface de rayonnement présente une superficie comprise dans la plage de 2, 0 mm² à 10 mm²,
- d'un agencement détecteur (40 ; 41) muni d'au moins deux éléments détecteurs (50, 60) réalisés de manière appropriée pour détecter le rayonnement de lumière ou le rayonnement de chaleur généré par la source de rayonnement (30),
- d'au moins deux éléments de filtre passe-bande (51, 61) disposés sur les au moins deux éléments détecteurs (50, 60),
- d'une unité de contrôle (9) pour commander un fonctionnement de la source de rayonnement (30) et pour détecter des signaux des au moins deux éléments détecteurs (50, 60),
dans lequel au moins l'un des deux éléments de filtre passe-bande (51, 61) est réalisé de manière optiquement transparente à un rayonnement infrarouge qui est absorbé par un gaz de mesure,
dans lequel au moins l'un des au moins deux éléments de filtre passe-bande (51, 61) est réalisé de manière optiquement transparente à un rayonnement infrarouge à un rayonnement qui n'est pas absorbé par le gaz de mesure,
dans lequel au moins l'un des deux éléments détecteurs (50, 60) est disposé avec au moins l'un des éléments de filtre passe-bande (51, 61), disposés sur les au moins deux éléments détecteurs (50, 60), dans un agencement angulaire (52, 62) ayant un angle (53, 63) compris dans une plage de 20° à 80° par rapport à l'axe médian (32) du rayonnement à travers la source de rayonnement (30), dans lequel chacun des au moins deux éléments détecteurs (50, 60) est disposé à une première distance I₁ (34, 34') par rapport à l'axe médian (32) passant entre les au moins deux agencements angulaires (52, 62) comprise dans une plage de 0,1 mm à 10,0 mm,
dans lequel chacun des au moins deux éléments de filtre passe-bande (51, 61), disposés sur les au moins deux éléments détecteurs (50, 60), est disposé à une deuxième distance I₂ (35) par rapport à l'axe médian (32) passant entre les au moins deux agencements angulaires (52, 62) comprise dans une plage de 0,1 mm à 10,0 mm,
dans lequel
(i) l'agencement détecteur (41) muni des au moins deux éléments détecteurs (50, 60) et des au moins deux éléments de filtre passe-bande (51, 61) disposés sur les au moins deux éléments détecteurs (50, 60) est disposé à l'opposé de la source de rayonnement (30) à une troisième distance I₃ (33) comprise dans une plage de 0,1 mm à 10,0 mm, la troisième distance I₃ (33) résultant comme une distance directement au niveau ou le long de l'axe médian (32) passant entre les au moins deux agencements angulaires (52, 62), la source de rayonnement (30) étant disposée de manière centrée sur l'axe médian (32) passant entre les aux moins deux agencements angulaires (52, 62) entre les au moins deux éléments détecteurs (50, 60) munis des au moins deux éléments de filtre passe-bande (51, 61) disposés sur les au moins deux éléments détecteurs (50, 60),
ou
(ii) l'agencement détecteur (40) muni des au moins deux éléments détecteurs (50, 60) et des au moins deux éléments de filtre passe-bande (51, 61) disposés sur les au moins deux éléments détecteurs (50, 60) est disposé du même côté à proximité de la source de rayonnement (30), dans lequel, à l'opposé de la source de rayonnement (30) et des au moins deux éléments détecteurs (50, 60) avec les au moins deux éléments de filtre passe-bande (51, 61) disposés sur les au moins deux éléments détecteurs (50, 60), au moins un élément (39) optiquement réfléchissant et à configuration plane est disposé à une troisième distance I_{3'} comprise dans une plage de 0,1 mm à 5,0 mm, la troisième distance I_{3'} résultant comme une distance directement au niveau ou le long de l'axe médian (32) passant entre les au moins deux agencements angulaires (52, 62) ;
et dans lequel la pompe est aménagée pour amener le débit volumique en continu au trajet optique de la source de rayonnement (30) à l'agencement détecteur (40; 41).

2. Dispositif d'analyse selon la revendication 1, dans lequel les éléments de filtre passe-bande (51, 61, 71, 81) sont configurés pour un filtrage optique de lumière infrarouge dans une bande passante d'une gamme de longueurs d'onde de 2,5 µm à 14 µm.

3. Dispositif d'analyse selon l'une quelconque des revendications précédentes, dans lequel les éléments détecteurs (50, 60) sont configurés sous forme de détecteurs pyroélectriques, de bolomètres, de détecteurs à semi-conducteur, de thermopiles ou d'éléments thermiques.

4. Dispositif d'analyse selon l'une quelconque des revendications précédentes, dans lequel l'agencement détecteur (40) présente plus de deux agencements angulaires (52, 62, 72, 72') munis d'éléments détecteurs et d'éléments de filtre passe-bande dans une disposition spatiale sous la forme de surfaces latérales d'un tronc de pyramide rectangulaire ou carré autour d'un centre (2).

5. Dispositif d'analyse selon l'une quelconque des revendications 1 à 4, dans lequel l'agencement détecteur (40, 41) réalise avec la source de rayonnement (30), et le cas échéant l'élément optiquement réfléchissant (39), un élément de guidage d'écoulement adapté pour guider le mélange de gaz respiratoire, de sorte que le mélange de gaz respiratoire traverse l'élément de guidage d'écoulement sous forme d'écoulement et la concentration de gaz dans l'écoulement peut être détectée.

6. Dispositif d'analyse selon l'une quelconque des revendications 1 à 4, comprenant un élément de guidage d'écoulement (100, 100'), adapté pour guider le mélange de gaz respiratoire, sur lequel l'agencement détecteur (40) et la source de rayonnement (30), et le cas échéant l'élément réfléchissant (39), sont disposés de telle sorte que le mélange de gaz respiratoire qui s'écoule substantiellement au milieu de l'élément de guidage d'écoulement (100) comme un écoulement principal est utilisé pour la mesure de concentration.

7. Dispositif (1) selon l'une quelconque des revendications 1 à 4, comprenant un élément de guidage d'écoulement (100") adapté pour guider le mélange de gaz respiratoire, dans lequel l'agencement détecteur (41) est réalisé avec la source de rayonnement (30), et le cas échéant l'élément réfléchissant (39), sous la forme d'un composant (97) disposé latéralement dans l'élément de guidage d'écoulement (100"), le composant (97) étant réalisé de telle sorte que le mélange de gaz respiratoire qui s'écoule substantiellement dans une zone marginale latérale de l'élément de guidage d'écoulement (100") dans un écoulement secondaire comme une partie d'un écoulement principal à travers le composant est utilisé pour la mesure de concentration.
